# EUROPÄISCHE PATENTSCHRIFT

(11) **EP 0 792 889 B1**
(45) Veröffentlichungstag und Bekanntmachung des Hinweises auf die Patenterteilung: **27.12.2000**
(21) Anmeldenummer: 97106769.9
(22) Anmeldetag: 08.08.1994
(51) Int. Cl.: C08B 37/18, C08L 101/00, C08L 3/00, C08L 1/00, C04B 24/38, C04B 35/00, D21H 17/24, D06M 15/03, A61K 47/36

(54) **Verwendung von Inulinderivaten**
Use of inulin derivatives
Application de dérivés d'inuline

(30) Priorität: 10.08.1993 DE 4326782
(43) Veröffentlichungstag der Anmeldung: 03.09.1997
(62) Teilanmeldung aus: 94112389.5
(73) Patentinhaber: SÜDZUCKER AKTIENGESELLSCHAFT MANNHEIM/OCHSENFURT, D-68165 Mannheim (DE)
(72) Erfinder: Kunz, Markwart, Dr., 67550 Worms (DE); Haji Begli, Alireza, Dr., 67305 Ramsen (DE)
(74) Vertreter: Schrell, Andreas, Dr.

(56) Entgegenhaltungen:
- EP-A- 0 539 235
- PATENT ABSTRACTS OF JAPAN vol. 013, no. 111 (C-577), 16.März 1989 & JP 63 287710 A (ICHIMARU PHARCOS CO LTD), 24.November 1988,

## Beschreibung

Gegenstand der vorliegenden Erfindung ist die Verwendung von Inulinderivaten.

Natürlich vorkommende Polysaccharide finden aufgrund ihrer funktionellen Eigenschaften Verwendung in den verschiedenen industriellen Bereichen wie z.B. Textil-, Papier-, Bau-, Keramik und Kunststoffindustrie. Dieser Verwendung geht zumeist eine anwendungsgerechte Derivatisierung voraus. Der besondere Vorteil solcher Verbindungen besteht darin, daß sie aus "nachwachsenden Rohstoffen" erzeugt werden und weitgehend biologisch abgebaut werden.

Voraussetzungen für eine breite Anwendung sind neben der leichten Verfügbarkeit und einem günstigen Preis die gleichbleibende Qualität und die Möglichkeit einer spezifischen und reproduzierbaren Derivatisierung.

Die bisher überlicherweise eingesetzten Polysaccharide, nämlich Cellulose und Stärke, sind zwar in jeder beliebigen Menge in hoher gleichbleibender Qualität zu einem günstigen Preis verfügbar, zeigen jedoch erhebliche Probleme bei der Derivatisierung.

Cellulose ist zwar ein linear einheitlich aus β-1-4-glucosidisch-verknüpften Anhydroglucoseeinheiten, aufgebautes Polymer; ihre Kristallinität macht sie jedoch gegenüber einer Derivatisierung äußerst widerstandsfähig. So sind Cellulosederivate nur unter schwierigen Reaktionsbedingungen, zum Teil unter Verwendung umweltgefährdender Chemikalien, erhältlich.

Auch der hohe Polymerisationsgrad der Cellulose und die damit verbundene Schwerlöslichkeit der Derivate begrenzen die Anwendbarkeit. Ein Abbau der Cellulose durch Enzyme oder Säuren führt zu sehr uneinheitlichen Bruchstücken, was wiederum die Derivatisierung erschwert.

Die zwar leichter zu derivatisierende Stärke besteht dagegen aus einem heterogenen System. Neben der linear aufgebauten Amylose mit einer -1-4-glucosidische Verknüpfung enthält sie eine Amylopektin genannte durch 1-6-glucosidische Bindungen verzweigte Komponente, deren Anteil in der Stärke außerdem bis zu 80 % schwanken kann. Da sich Amylose und Amylopektin bezüglich Struktur und Eigenschaften stark voneinander unterscheiden und in den verschiedenen Stärken in sehr variablen Anteilen vorkommen, ist eine gezielte Derivatisierung der Stärke nicht möglich.

Inulin, ein zur Gruppe der Fructane gehörendes Polysaccharid kommt in wirtschaftlich gewinnbaren Mengen in Teilen verschiedener Pflanzen wie z.B. Topinambur- und Dahlienknollen sowie in Zichorienwurzeln vor. Inulin besteht aus linear aufgebauten, β-2-1-verknüpften Ketten von im allgemeinen bis zu 50 β-O-Fructofuranosemolekülen, die als Abschluß jeweils eine -D-Glucopyranoseeinheit am reduzierenden Ende tragen. Die mittlere Kettenlänge und Molekulargewichtsverteilung sind sowohl von der Pflanzenart, als auch von der Wachstumsphase und der Aufbereitungsmethode abhängig. Mittlere Kettenlängen von 10 bis 25 sind üblich, wobei die Einzelmoleküle etwa 5 bis 50 Fructoseeinheiten aufweisen.

Obwohl das Inulin seit längerem bekannt ist, hat es noch keine breite Anwendung gefunden. Seine industrielle Anwendung beschränkt sich zur Zeit auf die unmittelbare Verwendung als Nahrungsmittelkomponente wie z.B. als Füllstoff oder Fettersatzstoff zur Herstellung von kalorienarmer Kost sowie zur Herstellung von Fructosesirupen bzw. Fructooligosacchariden mittels saurer oder enzymatischer Hydrolyse.

Es ist ferner nach E. Schacht et al in J. Controlled Release 1984, 1 (1), 33-46 bekannt, daß durch vorausgehende Oxidation eine Ankopplung von Dextran oder Inulin an Pharmazeutika wie Procainamid möglich ist. Gleichzeitig wird in dem Artikel die Aktivierung der genannten Polysaccharide mit Epichlorhydrin beschrieben. Da jedoch durch das als bifunktionelles Reagenz bekannte Epichlorhydrin das Polysaccharidgerüst je nach Reaktionsbedingung verzweigt bzw. vernetzt wird (Modified starches: properties and uses: O.B. Würzburg, (1986) S. 43 - 44), führt diese Umsetzung zu keinen einheitlichen Derivaten.

Aus der EP 0 539 235 A2 sind kalorienarme Nahrungsmittel wie Fettersatzstoffe bekannt, die dadurch hergestellt werden, daß man Polysaccharide, insbesondere Stärke und Cellulose, in Kohlenwasserstoffen wie Toluol bzw. Xylol suspendiert, mit wäßrigem Alkalihydroxid katalysiert, das zugesetzte Wasser destillativ entfernt und anschließend das aktivierte Polysaccharid bei hohem Druck und hoher Temperatur mit Alkylepoxiden veräthert. Die resultierenden Derivate werden anschließend mit C₈ - C₂₄ Fettsäuren verestert. Die hier erwähnten Polysaccharide sind in erster Linie Stärke und Cellulose, können jedoch auch Polydextrose, Xylan, Mannan oder ein nicht näher definiertes Inulin sein. Das hierbei zur Alkoxylierung angewandte Verfahren ist aus den US-PS'en Nr. 4.585.858 und 3.336.291 insbesondere für Stärke und Cellulose bekannt. Auch hierbei ergeben sich wegen des strukturellen Aufbaus der Polysaccharide erhebliche Probleme bei der Derivatisierung.

Aufgabe der Erfindung ist die Bereitstellung neuer Verwendungen von epoxidierten Inulinderivaten von gleichmäßiger Substitution. Zur Lösung dieser Aufgabe werden die Verwendungen gemäß des einzigen Anspruchs vorgeschlagen.

Überraschenderweise hat sich gezeigt, daß die Derivatisierung von Inulin aufgrund seiner chemischen Struktur, seiner Löslichkeit in Wasser sowie der relativ geringen Viskosität in wäßrigen Lösungen - im Gegensatz zu Stärke und Cellulose - in homogener Phase durchgeführt werden kann. Dadurch entfallen die typischen Probleme einer heterogenen Reaktionsführung; es wird eine gleichmäßige Verteilung der Reagenzien sowie eine bessere sterische Zugänglichkeit erreicht, was zu einer homogenen Verteilung der Substituenten im gewünschten Endprodukt führt.

Die erfindungsgemäß verwendete Verbindung wird hergestellt, indem man ein Inulin der Formel II 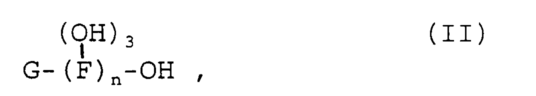 in der n die mittlere Kettenlänge definiert und eine Zahl von 10 bis 50, vorzugsweise > 15 und insbesondere > 20,
G eine Glycopyranosegruppe und
F eine Fructofuranosegruppe bedeuten, deren drei freie OH-Gruppen zusätzlich dargestellt sind,
in einer wäßrigen Lösung, in Gegenwart eines basischen Katalysators mit Epoxiden der allgemeinen Formel III 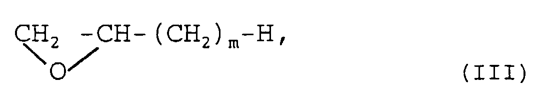 in der m eine ganze Zahl von 0 bis 10 bedeutet,
unter Bildung von Inulinderivaten der allgemeinen Formel veräthert: 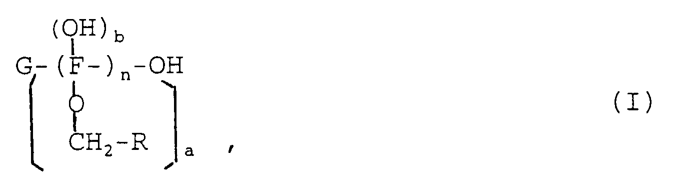 in welcher
R = -CH₂OH, -CHOH(CH₂)ₘ-CH₃,
m eine ganze Zahl von 0 bis 10,
n im Mittel eine Zahl von 10 bis 50,
a und b eine Zahl von 0 bis 3, wobei a+b=3 ist,
G eine Glucopyranosegruppe und
F eine Fructofuranosegruppe bedeuten.

Die Umsetzung wird mit Konzentrationen von 20 bis 50 Gew.-% bei einer Temperatur von 40 bis 100°C durchgeführt. Um ein Derivat mit dem gewünschten Substitutionsgrad herstellen zu können, genügt eine Veränderung des stöchiometrischen Verhältnisses der Reaktanten. Der Substitutionsgrad (DS) wird als das Verhältnis der Alkylgruppen zu den Fructoseeinheiten angegeben, wobei in der Regel mehrere Fructoseeinheiten je Inulinmolekül substituiert sind. Da jede Fructoseeinheit 3 freie Hydroxylgruppen aufweist, ist auch eine Mehrfachsubstitution möglich. Substitutionsgrade von 0,1 bis 2 und vorzugsweise von 0,5 - 1, sind für die meisten Anwendungen vorteilhaft.

Das aus Planzenteilen gewonnene sogenannte Rohinulin ist bei dem hier beschriebenen Verfahren direkt einsetzbar, jedoch sind die dabei erhaltenen Endprodukte bezüglich ihres Molekulargewichtes uneinheitlich. Es ist daher vorteilhaft, ein langkettiges Inulin, z.B. mit einer mittleren Kettenlänge von >15, insbesondere >20 Fructoseeinheiten einzusetzen, aus dem die kurzkettigen Bestandteile mit z.B. ≤ 10 bis 12 Fructoseeinheiten abgetrennt worden sind. Solche Produkte lassen sich gemäß dem in DE 43 16 425-A1 beschriebenen Verfahren herstellen. Alternativ lassen sich die niederen Ketten chromatographisch abtrennen, oder die längeren Ketten durch Fällung aus einer wäßrigen Lösung mit Alkohol erhalten.

Als geeignete Katalysatoren für die Umsetzung kommen Alkali- und Erdalkalihydroxide sowie andere für Verätherungsreaktionen bekannte Katalysatoren in Frage, wobei Natriumhydroxid und stark basische, OH-beladene Anionenaustauscher besonders bevorzugt sind. Die Einsatzmenge des Katalysators variiert in Abhängigkeit von der Reaktivität der eingesetzten Substituenten und beträgt im allgemeinen 2 bis 10 Gew.-% des vorgelegten Inulins.

Da sowohl die Reaktivität der Substituenten als auch deren Löslichkeit in Wasser mit steigender Alkylkettenlänge abnehmen, kann die Reaktionseffizienz dadurch gesteigert werden, daß man in Gegenwart einer geringen Menge eines Lösungsvermittlers wie Isopropanol arbeitet. Die Löslichkeit der Produkte mit langen Alkylketten kann zusätzlich gesteigert werden, wenn anstelle des nativen Inulins Inulinderivate eingesetzt werden, welche bereits teilweise mit kurzkettigen Epoxiden derivatisiert sind.

Die Umsetzung selbst kann in den dafür geeigneten Apparaturen unter Atmosphären durch bzw. bei erhöhtem Druck kontinuierlich oder auch diskontinuierlich durchgeführt werden. Die Reaktion ist in der Regel nach 6 bis 24 h beendet.

Bei der Verätherung mit wassermischbaren Epoxiden kann die Reaktion in einem geeigneten Reaktor, z.B. in einem Rohrreaktor unter Einsatz eines starkbasischen, OH-beladenen Anionenaustauschers anstelle von Alkali- bzw. Erdalkalihydroxiden unter Festbett-bzw. Wirbelbettbedingungen durchgeführt werden. Dabei wird eine klare 20 bis 40 gew.-%ige Inulinlösung durch einen auf 50 bis 70°C temperierten, mit Anionenaustauscherharz gefüllten Rohrreaktor gepumpt, und gleichzeitig wird das Reagenz über eine separate Leitung unter guter Durchmischung zudosiert.

Diese Arbeitsweise hat den Vorteil, daß eine Neutralisation des Reaktionsproduktes entfällt. Es ist jedoch empfehlenswert, das eingesetzte Inulin vorher zu entsalzen.

Wird die Reaktion mit Alkali- oder Erdalkalihydroxid katalysiert, empfiehlt es sich, das erhaltene Produkt anschließend zu neutralisieren. Das erfolgt zweckmäßigerweise mit verdünnter Salzsäure bzw. mit einem H⁺-beladenen schwachsauren Kationenaustauscher. Nach Trocknen eignen sich diese Produkte unmittelbar für eine Vielzahl von Anwendungen.

Darüber hinaus kann nach der Neutralisation das Derivat durch chromatographische Trennung von einem Ca⁺⁺-bzw. K⁺- oder Na⁺-beladenen, schwachvernetzten, starksauren Kationenaustauscherharz (z.B. Duolite (Wz) C204 von Rohm und Haas) gereinigt und durch geeignete Trocknungsmethoden, z.B. Gefrier- bzw. Sprühtrocknung, in trockene Form überführt werden.

Bei den so hergestellten Inulinderivaten handelt es sich um weiße geruchlose, kaltwasserlösliche Substanzen, die je nach Substitutionsgrad und Alkylkettenlänge des Substituenten interessante Eigenschaften, insbesondere hinsichtlich ihrer Löslichkeit, Oberflächenaktivität und Viskosität aufweisen.

Die alkoxylierten Derivate zeigen verbesserte Kaltwasserlöslichkeit gegenüber dem eingesetzten Inulin. Von den Derivaten können problemlos konzentrierte Lösungen, wie z.B. eine 50 Gew.%ige Lösung bei 25°C hergestellt werden.

Die erfindungsgemäß verwendeten, derart synthetisierten Inulinderivate zeigen in Abhängigkeit von der Temperatur und dem Substitionsgrad ein inverses Lösungsverhalten. Bei Temperaturen von 45 - 60°C flocken sie aus ihren wäßrigen Lösungen aus und gehen bei erneuter Abkühlung wieder in Lösung. Weiterhin sind sie in den üblichen org. Lösungsmitteln, wie z.B. MeOH, EtOH, DMF und DMSO löslich, wobei die Löslichkeit in protischen als auch in aprotischen Lösungsmitteln eine Korrelation zu den Strukturparametern wie Alkylkettenlänge und Substitionsgrad aufweist. Das Zahlenmittel des Molekulargewichtes (Mn) der synthesierten Inulinderivate liegt nach osmometrischer Bestimmung im Bereich von 3 000 bis 5 500 g/mol und die korrespondierenden Viskositätswerte der 3 Gew.%igen Lösungen der Inulinderivate bei 20°C zwischen 15 und 25 mPa.s.

Die derart hergestellten Inulinderivate eignen sich daher als
- polymere Weichmacher für Thermoplaste oder für unter Verwendung von Stärke und Stärkederivaten, Cellulose oder Cellulosederivaten hergestellte Folien,
- Hilfsstoffe für die Bau-, Keramik- und Papierindustrie,
- Emulgatoren und Stabilisatoren,
- Schlichtematerialien für die Textilindustrie,
- Trägermaterialien für pharmazeutische Wirkstoffe,
- Solubilisatoren für hydrophobe Arzneimittel und Kosmetika oder als
- gelbildende Polymere.

In den folgenden Beispielen wurde ein durch Alkoholfällung gereinigtes Rohinulin mit einer mittleren Kettenlänge von 18 bis 20 Fructoseeinheiten verwendet. Die Ausbeute bezieht sich jeweils auf die Summe der Ausgangsprodukte. Als Nebenprodukt entsteht jeweils das Alkylglykol durch Hydrolyse.

### Beispiel 1

20 g Trockensubstanz Inulin (0,123 mol) wurden in 60 ml 0,5 M NaOH (6 Gew.% auf Inulin TS) unter Erwärmen gelöst, in einen Rührautoklaven transferiert und dort mit 7,14 g (0,123 mol) Propylenoxid versetzt. Die Reaktion wurde dann bei 3 bar Druck (Stickstoff) und 70°C mit 800 x min⁻¹ Rührerdrehzahl durchgeführt. Nach 10 Stunden wurde der Autoklav entspannt und die entstandene Produktlösung zunächst mit HCl auf pH 6,5 - 7,0 neutralisiert. Eine Chromatographiesäule mit einem Na⁺-beladenen, schwachvernetzten, starksauren Kationenaustauscherharz (Duolite® C204) wurde vorbereitet und daran die neutralisierte Produktlösung bei 60°C getrennt. Die Propoxyinulin enthaltende Fraktion wurde gesammelt und gefriergetrocknet.

| | |
|---|---|
| Ausbeute | 92 % |
| Substitutionsgrad (DS) | 0,66 |
| Oberflächenspannung σ einer 1 %-igen Lösung | 62 mN.m⁻¹ |

### Beispiel 2

650 g einer 30 gew.-%igen Inulinlösung (≅ 1,2 mol) wurden mit 9,8 g (5 Gew.% auf Inulin-TS) Natriumhydroxid versetzt und in einen auf 55°C temperierten, mit Intensivkühler bestückten Rohrreaktor im Kreislauf gepumpt. In die zirkulierende Inulinlösung wurden innerhalb 60 min 67 g (≅ 1,2 mol) Propylenoxid kontinuierlich zudosiert. Die Lösung wurde für weitere 6 h bei 55°C umgepumpt.

Die erhaltene Produktlösung wurde mit Hilfe eines H⁺-beladenen starksauren Kationenaustauschers neutralisiert. Nach Filtration und Eindampfung auf 40 % Trockensubstanzgehalt wurde die Lösung über eine mit Chromatographieharz (Lewatit® TSW 40-Ca⁺⁺) gefüllte Trennsäule bei 60°C getrennt. Die Propoxyinulin enthaltende Fraktion wurde gesammelt und daraus das Propoxyinulin durch Sprühtrocknung als weißes Pulver isoliert.

| | |
|---|---|
| Ausbeute | 85,7 % |
| Substitutionsgrad (DS) | 0,71 |
| Oberflächenspannung σ einer 0,1 %-igen Lösung | 64 mN.m⁻¹ |

### Beispiel 3

256 g Inulin Trockensubstanz (1,58 mol) wurden in 592 g entmineralisiertem Wasser durch Erwärmen gelöst und in einen mit Intensivkühler und Tropftrichter bestückten, auf 55°C temperierten Rührkessel gegeben und zunächst mit 500 ml eines OH⁻-beladenen starkbasischen Anionenaustauschers (Amberlite® IRA 900) versetzt. Danach wurden 91,82 g (1,58 mol) Propylenoxid innerhalb von 4 Stunden der Lösung zugetropft. Danach wurde der Reaktionsansatz weitere 12 h bei 55°C gerührt.

Dann wurde das Anionenaustauscherharz abfiltriert, das Filtrat mit Hilfe eines H⁺-beladenen starksauren Kationenaustauschers neutralisiert und chromatographisch getrennt. Durch Sprühtrocknung der Propoxyinulin enthaltenden Fraktion wurde das Propoxyinulin als weißes Pulver gewonnen.

| | |
|---|---|
| Ausbeute | 85 % |
| Substitionsgrad (DS) | 0,5 |
| Oberflächenspannung σ einer 0,5 %-igen Lösung | 58 mN.m⁻¹ |

### Beispiel 4

In einen mit einem Siebboden versehenen und auf 55°C temperierten Rohrreaktor wurden zunächst 250 ml eines OH⁻-beladenen starkbasischen Anionenaustauschers (Amberlite® IRA 900) vorgelegt. Dann wurden 480 g einer 30 gew.%-igen Inulinlösung (144 g Trockensubstanz ≅ 0,89 mol) im Kreislauf von unten nach oben durch den Reaktor gepumpt. Die Fließgeschwindigkeit wurde dabei so gewählt, daß das Harzbett sich in ein Wirbelbett verwandelte. Nun wurden während 60 Minuten 51,6 g (0,89 mol) Propylenoxid in den Inulin-Kreislauf hineindosiert. Nach weiteren 6 h Reaktionszeit wurde die Produktlösung vom Katalysator abgetrennt, neutralisiert und einer chromatographischen Trennung unterworfen. Die Propoxyinulin enthaltende Fraktion wurde sprühgetrocknet und so das Propoxyinulin in trockner Form isoliert.

| | |
|---|---|
| Ausbeute | 87,3 % |
| Substitutionsgrad (DS) | 0,36 |
| Oberflächenspannung σ einer 0,5 %-igen Lösung | 59 mN.m⁻¹ |

### Beispiel 5

In einen mit einem Siebboden versehenen und auf 55°C temperierten Rohrreaktor wurden zunächst 250 ml eines OH⁻-beladenen starkbasischen Anionenaustauschers (Amberlite® IRA 900) vorgelegt. Dann wurden 480 g einer 30 gew.-%igen Inulinlösung (144 g Trockensubstanz = 0,89 mol) durch den Reaktor gepumpt. Die Fließgeschwindigkeit wurde dabei so gewählt, daß eine Gesamtkontaktigkeit von 8 Stunden eingehalten werden konnte. Gleichzeitig wurden dem Flüssigkeitsstrom vor dem Reaktor insgesamt 51,6 g (0,89 mol) Propylenoxid verteilt auf die gesamte Kontaktigkeit zudosiert. Die vom Reaktor ablaufende Produktlösung wurde neutralisiert und daraus das Propoxyinulin durch chromatographische Trennung mit anschließender Sprühtrocknung der relevanten Fraktion als weißes Pulver isoliert.

| | |
|---|---|
| Ausbeute | 87 % |
| DS | 0,38 |
| Oberflächenspannung σ einer 0,5 %-igen Lösung | 58 mN.m⁻¹ |

### Beispiel 6

60 g propoxiiiertes Inulin (mit DS = 1,33) wurden in 200 ml Wasser gelöst, mit 3,0 g NaOH und 68 g Epoxydodecan versetzt und im Autoklaven unter 3 bar Druck (N₂), 80°C und bei Rührerdrehzahl 900 x min⁻¹ für 16 h reagieren gelassen. Dann wurde die Reaktionsmischung mit n-Hexan extrahiert, die wäßrige Phase neutralisiert und daraus durch chromatographische Trennung und Sprühtrocknung das Dodecyloxyinulin gewonnen.

| | |
|---|---|
| Ausbeute | 70 % |
| Oberflächenspannung σ einer 0,1 %-igen Lösung | 29 mN.m⁻¹ |

### Beispiel 7

| | |
|---|---|
| Ansatz | 6 g TS Inulin in Form einer 30 gew.%-igen Lösung |
| | 0,3 g NaOH |
| | 4 g Epoxybutan |
| Temperatur | 55°C |
| Reaktionszeit | 16 h |
| Ausbeute | 92 % |
| DS | 0,6 |
| Oberflächenspannung σ einer 0,1 %-igen Lösung | 49 mN.m⁻¹ |

### Beispiel 8

| | |
|---|---|
| Ansatz | 30 g TS Inulin in Form einer 30 gew.%-igen Lösung |
| | 65 ml Amberlite® IRA 900 CH- |
| | 20 g Epoxybutan |
| Temperatur | 55°C |
| Reaktionszeit | 16 h |
| Ausbeute | 78 % |
| Oberflächenspannung σ einer 0,1 %-igen Lösung | 47 mN.m⁻¹ |
| DS | 0,56 |

### Beispiel 9

| | |
|---|---|
| Ansatz | 30 g TS Inulin in Form einer 30 gew%igen Lösung |
| | 65 ml Amberlite® IRA 900 CH- |
| | 23,2 g 1,2-Epoxyhexan |
| Temperatur | 60 °C |
| Reaktionszeit | 16 h |
| Ausbeute | 73 % |
| Oberflächenspannung σ einer 0,1 %-igen Lösung | 43 mN.m^{-s} |

### Beispiel 10

| | |
|---|---|
| Ansatz | 30 g TS Inulin in Form einer 30 gew.%-igen Lösung |
| | 1,5 g NaOH |
| | 23,2 g 1,2-Epoxyhexan |
| Temperatur | 55°C |
| Reaktionszeit | 16 h |
| Ausbeute | 78 % |
| DS | |
| Oberflächenspannung σ einer 0,1 %-igen Lösung | 41 mN.m⁻¹ |

### Beispiel 11

Um zu zeigen, daß durch den Einsatz der derart hergestellten Inulinderivate die Konsistenz von hydraulischen Bindesystemen positiv beeinflußt wird, wurde ein Kalkmörtel der folgenden Zusammensetzung hergestellt:

### Beispiel

- 50 %: beta-Kalziumsulfat Halbhydrat
- 20 %: Kalkhydrat
- 29 %: Kalksteinsand (0 - 1 mm)
- 0,8 %: Leichtfüllstoff
- 0,02 %: Stärkeether
- 0,02 %: Luftporenteilchen
- 0,04 %: Verzögerer
- 0,17 %: Celluloseether

Es wurden entsprechende Abmischungen mit 0,01 bis 0,1 Gew.% Alkoxyinulinderivaten entsprechend Beispiele 2 und 7 hergestellt und mit dem Grundmörtel verglichen, wobei festgestellt wurde, daß anwendungstechnische Eigenschaften, speziell die Verarbeitbarkeit, das Wasserrückhaltevermögen und die konsistenzgebende Wirkung der gebräuchlichen Verdicker verbessert werden.

### Beispiel 12

Es wurde ein Textil-Schlichtemittel der folgenden Zusammensetzung hergestellt:
- 51 %: Schlichtstärke
- 17 %: PVA
- 30 %: Alkoxyinulinderivat
- 2 %: Schlichtewachs

Bei dieser Rezeptur wurden jeweils die Alkoxyinulinderivate gemäß Beispielen 3, 7 und 8 eingesetzt. Hierbei zeigte sich, daß die Konsistenz der abgewandelten Schlichten im Vergleich zu üblichen Textilschlichten, die auf synthetischer Basis (PVA, Acrylat), Stärkebasis oder deren Mischungen aufgebaut sind und deren Elastizität, Klebkraft, Auswaschbarkeit, Laufeigenschaften und Web-Nutzeffekt verbessert wird.

### Beispiel 13

Bei der Leimung von Papier wurde eine Mischung der folgenden Zusammensetzung verwendet:
- 50 bis 100 g/l: Stärke
- 1 bis 5 g/l: Leimungsmittel (Acrylat/Methacrylat)
- 10 bis 20 g/l: Alkoxyinulinderivate

Es zeigte sich, daß durch den Einsatz der Inulinderivate gemäß Beispiel 2, 6, 8 und 10 in Verbindung mit Acrylat-Methacrylatprodukten und Stärke die Oberflächeneigenschaften, wie Reißlänge, Spaltfestigkeit, Weißgrad, Oberflächenglätte, Bedruckbarkeit von Papier verbessert werden konnten.

Weiterhin zeigen die alkoxylierten Inulinderivate gute Schutzkolloid- bzw. Emulgator-Eigenschaften bei der Herstellung von Kunststoffdispersionen insbesondere bei den Polyvinylacetat- und ihre Copolymerisationsdispersionen.

Aufgrund ihrer guten Eigenschaften (wie z.B. hydrophil - hydrophoben Charakter, Viskosität, Molmasse etc.) werden die Inulinderivate besonders gut an der Oberfläche der Latexpartikel adsorbiert. Die so gebildete Schutzschicht verhindert das Koagulieren der Dispersion bei der Polymerisation und stabilisiert die fertige Dispersion bei der mechanischen Beanspruchung.

Zur Prüfung des Solubilisationsvermögens alkoxylierter Inuline für nicht- bzw. schlecht wasserlösliche Substanzen wurden beispielhaft 2 Chromophore (Antracen als UV-aktive Substanz und der im sichtbaren Bereich absorbierende, wasserunlösliche Azofarbstoff Sudanrot B) in wäßrigen Lösungen der Inulinderivate dispergiert und die Extinktion bei jeweils charakteristischen Wellenlängen (Antracen: λ = 371 bzw. λ = 380 nm, Sudanrot B: λ = 516 nm) unter Variation der Konzentrationen der Inulinderivate ermittelt. Zum Vergleich wurden wäßrige Dispersionen der beiden Chromophoren ohne Zusatz der Inulinderivate hergestellt und vermessen.

Während für die Vergleichslösungen (ohne Inulinderivate) keine Extinktionen bei den charakteristischen Wellenlängen zu beobachten waren, konnten bei den Lösungen der Inulinderivate signifikante Extinktionen bestimmt werden. Die Solubilisationskapazität steigt mit zunehmender Konzentration der Inulinderivate, Länge der Alkoxylgruppe und der Substitutionsgerade.

Diese Untersuchungen zeigen, daß Alkoxyinuline geeignet sind, als Carrier bzw. Stabilisator für schlechte bzw. wasserunlösliche Substanzen zu fungieren (Zusatzstoffe in Kosmetika, Pharmazeutika "Prodrugs", "drug targeting", "retarded drug release", "biocompatible drug carrier").

## Patentansprüche

1. Verwendung von Inulinderivaten der allgemeinen Formel I 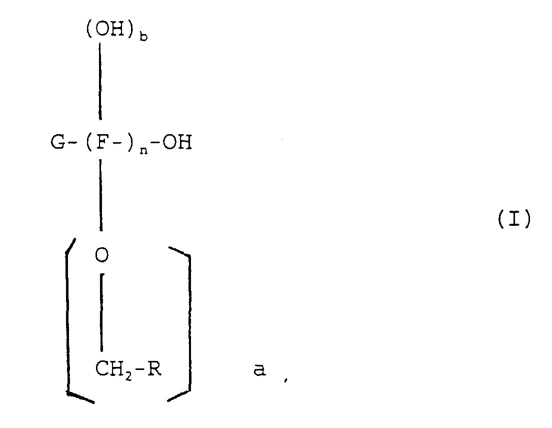 in welcher
R = CH₂OH, - CHOH - (CH₂)ₘ- CH₃,
m eine ganze Zahl von 0 bis 10,
n im Mittel eine Zahl von 10 bis 50 und vorzugsweise von >15,
a und b eine Zahl von 0 bis 3, wobei a+b=3 ist,
G eine Glucosepyranosegruppe und
F eine Fructofuranosegruppe bedeuten,
oder Gemischen dieser mit verschiedenen - 0 (CH₂)-R Gruppen als
a) polymere Weichmacher für Thermoplaste oder für unter Verwendung von Stärke und Stärkederivaten, Cellulose oder Cellulosederivaten hergestellten Folien,
b) Hilfsstoffe für die Bau-, Keramik- und Papierindustrie,
c) Emulgatoren und Stabilisatoren,
d) Schlichtematerialien für die Textilindustrie,
e) Trägermaterialien für pharmazeutische Wirkstoffe,
f) Solubilisatoren für hydrophobe Arzneimittel und Kosmetika oder als
g) gelbildende Polymere.

## Claims

1. Use of inulin derivatives of the general formula I 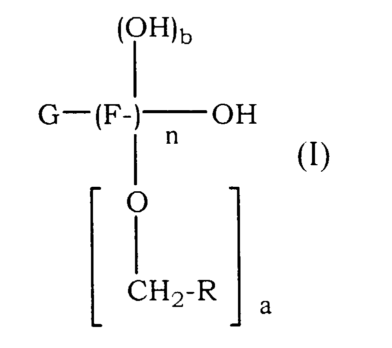 wherein
R = CH₂OH, -CHOH-(CH₂)ₘ-CH₃,
m is a whole number from 0 to 10,
n is on average a number from 10 to 50 and preferably > 15,
a and b are a number from 0 to 3, where a + b = 3,
G is a glucose-pyranose group, and
F is a fructofuranose group,
or mixtures of these with different [-O-CH₂-R] groups as
a) polymeric plasticisers for thermoplastics or for films manufactured using starch and starch derivatives, cellulose or cellulose derivatives,
b) auxiliary agents for the building, ceramics and paper industries,
c) emulsifiers and stabilisers,
d) sizing materials for the textile industry,
e) carrier materials for pharmaceutical active substances,
f) solubilisers for hydrophobic pharmaceutical preparations and cosmetics, or as
g) gel-forming polymers.

## Revendications

1. Utilisation de dérivés d'inuline correspondant à la formule générale I 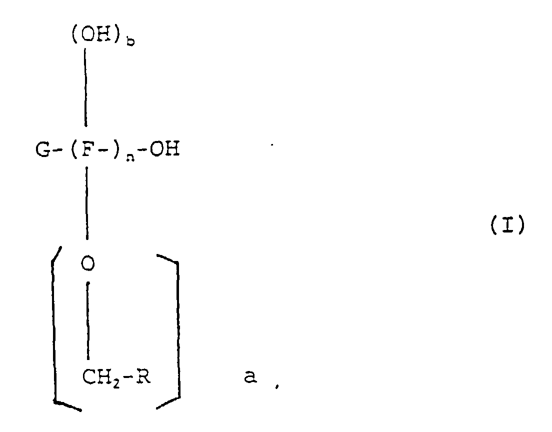 dans laquelle
R = CH₂OH, - CHOH - (CH₂)ₘ - CH₃,
m est un nombre entier compris entre 0 et 10,
n est en moyenne un nombre compris entre 10 et 50 et de préférence > 15,
a et b sont un nombre compris entre 0 et 3, avec a + b = 3,
G est un groupe glucopyranose, et
F est un groupe fructofuranose,
ou de mélanges de ceux-ci avec différents groupes - 0 (CH₂) - R en tant que
a) plastifiants polymères pour matières thermoplastiques ou pour feuilles fabriquées en utilisant des amidons et des dérivés d'amidon, de la cellulose ou des dérivés de cellulose,
b) agents auxiliaires pour l'industrie du bâtiment, de la céramique et du papier,
c) agents émulsifiants et agents stabilisants,
d) matériaux d'encollage pour l'industrie textile,
e) substances de support pour principes actifs pharmaceutiques,
f) agents de solubilisation pour médicaments et produits cosmétiques hydrophobes, ou en tant que
g) polymères gélifiants.
